# EUROPEAN PATENT SPECIFICATION

(11) **EP 2 395 915 B1**
(45) Date of publication and mention of the grant of the patent: **16.01.2019**
(21) Application number: 10741480.7
(22) Date of filing: 08.02.2010
(51) Int. Cl.: A61B 5/097, G01N 33/497, A61B 5/00

(54) **BREATH ANALYSIS**
ATMUNGSANALYSE
ANALYSE RESPIRATOIRE

(30) Priority: 10.02.2009 SE 0900162
(43) Date of publication of application: 21.12.2011
(73) Proprietor: Hok Instrument AB, 721 31 Vasteras (SE)
(72) Inventor: HÖK, Bertil, S-723 44 Västerås (SE)
(74) Representative: Brann AB
(86) International application number: PCT/SE2010/050146
(87) International publication number: WO 2010/093317

(56) References cited:
- EP-A2- 1 688 741
- EP-A2- 1 688 741
- WO-A1-01/08554
- WO-A1-01/08554
- WO-A1-01/28416
- WO-A1-95/26889
- WO-A1-95/26889
- DE-A1- 4 127 599
- US-A- 6 123 674
- US-A- 6 123 674
- US-A1- 2008 061 238

## Description

The present invention is concerned with broadening the present applicability of breath analysis for the detection of volatile substances, e g ethyl alcohol in expired breath. The present applicability has serious limitations with respect to the degree of cooperation, and the physiological capabilities required by the test person. The present invention enables breath analysis from incapable persons, by which is meant persons unable to cooperate by providing forced expiration, and persons with a physical or psychological handicap with decreased lung capacity.

At present, determination of the alcohol concentration of an unconscious patient within the health care system can only be performed by laboratory analysis of blood samples, which is costly and often takes more than one hour. Breath analysis is not practical in unconscious patients, since alcometers according to the state of the art require active cooperation from the test person, including forced expiration of a sample volume of 0.7-1.2 liters, using a tightly fitting mouthpiece.

Swedish emergency care receives approximately 50 000 cases of unconscious patients annually, mainly patients with stroke, epilepsy, cardiac infarct, coma, trauma or intoxication from alcohol or drugs. According to public statistics, approximately 250 persons die each year in Sweden as a consequence of acute alcohol intoxication, and this is also the most common cause of intoxication.

The absence of methods and equipment for objective and rapid determination of alcohol influence may lead to erroneous treatment or none at all when symptoms are difficult to interpret - in the worst case this may be a question of life or death. Access to rapid alcohol determination within emergency health care would reduce the risk that cardiac infarct, stroke, coma, or trauma, are mistaken for more easily handled alcohol intoxication.

Ambulance operators, rescue teams, clinicians at emergency care centers, and others would benefit from improved method and apparatus for breath analysis. Applications include sobriety tests performed by the police force, and alcolocks for the prevention of drunk driving. In these applications, it is generally required to deliver a vital capacity forced expiration, which is not only time consuming, but also requires a great deal of effort from the test person. Furthermore, the breath analyzer according to the state of the art is generally equipped with a replaceable mouthpiece for hygienic reasons. The test person is assumed to be capable of providing an airtight seal to the mouthpiece.

The prospective equipment and method for expanded applicability would have to meet the following requirements: Sampling and analysis should be basically independent of the test person's lung capacity, i e should require minimum sample volume and flow, and should be applicable both at spontaneous and assisted ventilation. An unconscious or handicapped person may exhibit significantly reduced tidal volume, i e the volume of each breath, compared to the normal value 500 ml of a passive breath. Furthermore, the demands are high with respect to easy operation, speed, accuracy, selectivity (identification of actual substances) reliability, protection against infection and environmental durability.

WO 01 /08554 discloses an indirect calorimeter for measuring the metabolic rate of a subject is provided, which includes a respiratory calorimeter (10) configured to be supported in contact with the subject, passing inhaled and exhaled gases as the subject breathes. A flow pathway (22) is operable to receive and pass inhaled and exhaled gases having a first end in fluid communication with the respiratory connector (20) and a second end is in fluid communication with a source and sink for respiratory gases. A flow meter generates electrical signals as a function of the instantaneous flow volume of inhaled and exhaled gases passing through the flow pathway. A component gas concentration sensor (84) generates electrical signals as a function of the instantaneous fraction of a predetermined component gas in the exhaled gases as the gases pass through the flow pathway. As the subject breathes through the calorimeter (10), a computation unit (96) receives electrical signals from the flow meter and the component gas concentration sensor (84), calculating at least one respiratory parameter.

US 2008/061238 discloses a system for the detection and analysis of at least one volatile substance in breath samples of a subject, including measurement of a reference substance, such as carbon dioxide or water vapour.

The present invention fulfills the requirements expressed above. Sampling can be performed, basically without physical contact by positioning a receptor designed for the purpose near the person's mouth and nose, however without sealing the opening, as is the case with mouthpieces according to prior art. The receptor according to the invention preferably exhibits a concentration preserving effect, without adding significant resistance to the respiratory airflow. The sample volume is less than 50 ml, i e a tenth of a normal passive breath. Determination of the concentration of ethyl alcohol or other volatile organic substance takes place directly in a measuring cell connected to the receptor. Air intake to the measuring cell is advantageously performed actively by means of a pump.

The concentration preserving effect of the receptor is based on its shape, including an inlet opening which, when sampling, is held in adjacent to the test person's mouth and nose. The area of the inlet opening is typically larger than the cross section of the mouth or nose openings of the test person. Thereby it will effectively collect the jet-like expiratory flow. The receptor is typically shaped like a cup, mug, funnel or scoop, with a partly confined inner volume which is preferably much smaller than the test person's tidal volume. The incident expiratory flow fills the inner volume, thereby evacuating the already present ambient air. The flow is distributed into one branch flowing into the measuring cell where the breath analysis takes place, and another branch which is redirected to the inlet opening. This reflowing branch forms a protective curtain, preventing the incident flow from mixing with ambient air, and will thus provide the effect of concentration preservation, i e the breath sample will not be diluted with ambient air.

By the concentration preserving effect, the substance concentration within the measuring cell will approach that of the inner airways, and the deviation may be considered insignificant compared with other error sources.

An important property of the present invention is the determination of a physiological reference substance, e g carbon dioxide, which occurs simultaneous with the alcohol determination. The concentration of the reference substance within the inner airways can be considered known, and therefore a measured value lower than normal is a sign that the sample has been diluted with ambient air. The determination of the reference substance in relation to the normal value is thus a quality measure of the analysis. The normal partial pressure of carbon dioxide is 4.8 kPa. Alternatively, water vapor can be used as physiological reference, with a normal value of 45 mg/l.

The simultaneous determination of a physiological reference means increased accuracy, minimization of the risk of false negative output, and simplified operation. The positioning of the receptor becomes less critical and the need for tight seal to the mouth or nose is eliminated. Further is obtained a univocal and permanent quality measure of the alcohol reading, or other unknown substance.

Before sampling, the measuring cell is automatically zeroed, followed by readiness indication. The analysis results are preferably presented within five seconds after finished sampling. Air flow through the measuring cell is only taking place at sampling and zeroing, resulting in full operating control of the measuring procedure. This is important for the reliability and environmental durability. The risk that the measuring cell will be exposed to destructive environmental influence is minimal.

Infrared (IR) spectroscopy is preferably used as measuring principle for both the unknown and the reference substances. The measuring cell is trans-illuminated by radiation from a broadband IR emitter hitting IR detectors with band pass filters for selected transmission bands. Ethyl alcohol is preferably detected as a decreasing transmittance in the wavelength range 3.3-3.5 µm compared to the reference level measured in clean air at zeroing. Carbon dioxide has a corresponding dip of transmission, or absorption peak, at 4.2-4.3 µm, and water at 2.5-2.8 µm.

In order to meet the requirements of accuracy the measuring cell should have a long transmission path for the IR radiation. The conflicting requirement of small overall physical size can be overcome by using an arrangement of multiple concave mirrors.

By IR spectroscopy it is possible both to identify and quantify all substances with absorption peaks in the actual wavelength range. IR spectroscopy has a fundamental advantage compared to other sensor principles spectroscopy with respect to reliability. On every measuring occasion it is possible to evaluate the stability of the baseline by comparing it to previous measurements. Thereby full control can be obtained of variations over time of the IR source, reflecting surfaces, filters, and detectors. Moreover, the sensitivity of the method is basically time independent, as opposed to sensors based on catalysis. The need for periodic calibration is therefore eliminated.

The detailed characteristics of the invention will be described in the following text relating to the drawings.
Figure 1 shows a flow chart of the elements of an embodiment of the method according to the invention.
Figure 2 shows a block diagram of a preferred embodiment of the apparatus according to the invention.
Figure 3 shows schematically a preferred embodiment of the receptor according to the invention, and the relevant air flows.

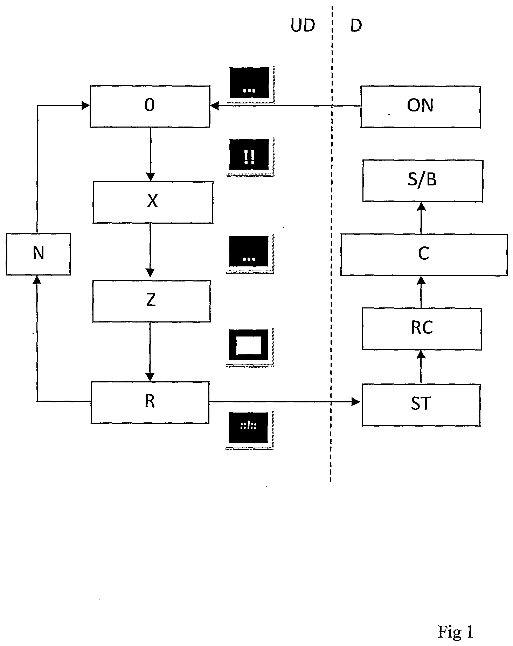

The flow chart of figure 1 shows ten different states, each state being illustrated with a box. The ten states are divided into two categories, one marked "UD" referring to active states with respect to the actual breath analysis, whereas "D" are passive states between occasions of operation. The abbreviations refer to "undocked" and "docked". The breath analyzer according to the invention includes a docking station for the handheld unit. In the active states, the handheld unit is undocked, and vice versa.

In a typical procedure according to the invention the apparatus is transferred from inactivated to active state category by moving the hand unit from the docking station, illustrated by the signature "ON", Then automatic zeroing and function test of the measuring cell occurs at state "0", while the display of the hand unit indicates a waiting condition, by the signature "..." as illustrated in figure 1.

At zeroing ambient air is pumped into the measuring cell, and its zero level will therefore be related to ambient air at present air composition, which is basically zero both for organic substances and carbon dioxide. The function test is related to the absolute value of the measuring variables, in which the deviation from previous measurement, as well as baseline drift shall be within certain tolerances to be approved. Otherwise error indication will follow.

When zeroing has been performed, ready indication is provided by the signature "!", and sampling "X" starts. The operator is then positioning the receptor and hand unit adjacent to the test person's mouth and nose and starts sampling by pressing a start button. Then air is pumped via the receptor through the measuring cell. Sampling and pumping continues until a certain CO₂ concentration is reached or longer. The operator may also choose to stop the sampling manually. Preferably, the sampling, analysis and signal presentation occurs in real time.

The analysis state "Z" may occur simultaneously or slightly after sampling, indicated by different signatures "..." and "□". The results of the analysis are thereafter presented on the display either graphically, numerically or in other form.

After result presentation the operator may choose to return the hand unit to the docking station or carry out a new measurement "N" whereby the procedure is repeated from zeroing and further. At docking a more extensive function test "ST" is performed, is performed together with charging of the battery of the hand unit "RC". Finally the apparatus is transferred into a sleeping state, "S/B".

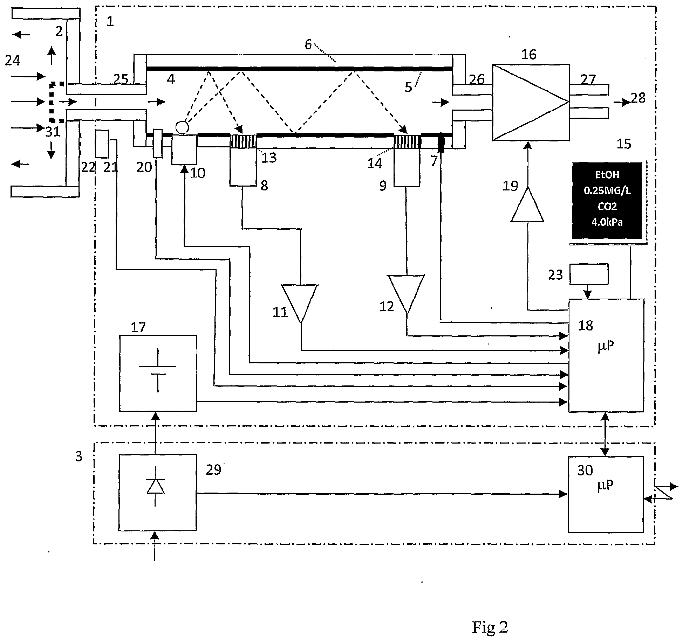

Figure 2 shows a block diagram of a preferred embodiment of the apparatus according to the invention. Basically the apparatus consists of three replaceable units, the receptor 2, the hand unit 1 and the docking station 3. Replacement of the receptor 2 from the hand unit 1 is motivated for hygienic reasons and the requirement of protection against infection. The receptor 2 may easily be contaminated at its position close to the mouth and nose of the test person, and should therefore be exchanged or cleaned before sampling from another test person. Replacement of the hand unit 1with respect to the docking station 3 is motivated by the fact that the hand unit should be rapidly taken from sleeping state without time consuming function tests. The requirement of a reliable voltage supply is another reason.

The receptor 2 is typically shaped like a cup, mug, funnel or scoop, with a larger inlet opening 24 which is larger than the mouth and nose openings of the test person, and when directed towards them at sampling will effectively receive the expiratory air flow which typically forms a jet. Inspiratory air flow, on the other hand, can take more curved paths.

The shape of the receptor 2 defines an inner volume which may be 10-100 ml, depending on the actual application. There is also a smaller inner opening connecting to the hand unit 1, and its measuring cell 4. Preferably, there is also an element 31 for separation of liquid drops and particles. Furthermore, the receptor 2 advantageously includes a mechanical support element for fixation to a body part, e g the nose and chin tips. It may also include means for connecting to a face mask or other aids for assisted breathing. The details of these implementations are not included in figure 2.

The inlet opening 24 of the receptor 2 has a cross section area larger than the corresponding areas of nose and mouth openings of a typical test person. The jet-like air stream from the mouth or nose of the test person with good margin will be received at the inner wall of the receptor 2 essentially without loss, and without requiring an accurate position control. Incident air flow is shown in figure 2 as three arrows pointing in the right direction. When the jet hits the inner wall of the receptor 2 it will be distributed into one branch which via the inner opening of the receptor 2 passes through the measuring cell and another, expanding branch that first moves from the centre and thereafter gives rise to a reverse flow compared to the incident one. The flow reflection or reflow emanates from the side wall of the receptor which at least partly prevents further radial expansion. The reflow is the origin of the earlier mentioned, concentration preserving effect by protecting the breath sample from being mixed with ambient air.

The hand unit 1 includes a measuring cell 4 designed for IR spectroscopic analysis of gas within the inner volume of the measuring cell. From the IR source 10 electromagnetic radiation is emitted at a relatively broad wavelength range, typically 2-6 µm, by black body radiation. Preferably the IR source is modulated at 2 Hz repetition frequency or higher in order to avoid baseline drift of the system. The measuring cell 4 is enclosed by molded walls 6 having highly reflecting surfaces of gold or aluminium with a reflection coefficient of 0.98 or higher. By multiple reflections against focusing surfaces, the demands on large aperture and long optical path may be combined with small physical dimensions of the measuring cell 4. Typical outer dimensions are 40 x 50 x 5 mm, with an inner volume of 5 ml.

The IR detectors 8, 9 are located at suitable positions in order to receive radiation from the IR source 10. The detector 8 is preferably positioned with a shorter optical path to the IR source 10, and adapted to the absorption band of CO₂ at 4.2-4.3 µm, alternatively water vapor at 2,5-2,8 µm. In front of the detector is an interference filter 13 of band pass type, adapted to precisely this wavelength range. Correspondingly, the detector 9 is adapted to detection of organics substances, e g ethyl alcohol, at the wavelength range 3.3-3.5 µm, and is therefore positioned with a longer optical path using an interference filter 14. With an optical path of 20 cm, a resolution of 0.02 mg/l ethyl alcohol is obtained. Higher resolution may be obtained by increasing the optical path, alternatively using a detector 9, and amplifier 12 with lower noise.

In a preferred embodiment of the invention it is possible to stop the inflow of air into the measuring cell at sampling and zeroing. At sampling this can take place when the CO₂ concentration has reached a certain threshold value. Absence of flow will minimize disturbances and noise caused by air movement within the measuring cell 4. Thereby maximum resolution can be obtained for the determination of volatile organic substances. When there is a demand for identification of organic substances, the detector 9 may include a multiple set of filters 14 in the wavelength range 3.0 to 3.6 µm. By analyzing the relative fractions of the signals within this range, it is possible to identify individual substances, due to the fact that their molecular structure gives rise to individually different fine structure of the absorption peak.

The filters 13, 14 may in an alternative embodiment be replaced by other dispersive elements for multi variable analysis, e g diffraction gratings, which may be advantageous due to their low fabrication cost.

The transport of the breath sample via the receptor 2 through the measuring cell 4 is preferably performed actively by a pump 16, via tubing 25, 26, 27 to the outlet opening 28 to ambient air. The pump 16 is advantageously closed when not activated, e g by embedded non-return valves. The pump 16 may be peristaltic or using membrane, centrifuge or cog wheels. Typically, the volume flow is 1-10 ml per second. Air flow to the measuring cell may preferably be closed at all times except during zeroing and sampling in order to protect the measuring cell 4 from environmental influence when the apparatus is in a sleeping or storage state.

The measuring cell preferably includes a heating arrangement 7 by which the reflecting surfaces 5 are heated to body temperature or above. The purpose of the arrangement 7 is to avoid condensation of water droplets on the reflecting surfaces. Preferably, a pressure transducer 20 is used for monitoring the pressure drop resulting from activation of the pump. Thus it is possible to detect whether or not the receptor 2 is correctly connected. Alternatively, a dedicated sensor arrangement 21, 22 is used for identification and detection of presence of the receptor 2.

The signals from the IR-detectors 8, 9, the pressure transducer 20, the sensor 21 and the start button 23 are taken via amplifiers 11, 12 to a microprocessor 18, in which signal processing takes place according to a preprogrammed sequence governed by the pattern described in the flow chart of figure 1. The microprocessor 18 has capacity to execute analog to digital conversion and to emit control signals to the IR source 10, the heating arrangement 7, the pump 16 via buffer stage 19, and the display 15.

The display 15 presents information about the results of the analysis, and the various states of the system by graphics, color or alphanumerical characters, preferably visible also at unfavorable illumination conditions, and observation angles.

The hand unit 1 is powered by a rechargeable battery 17, and its condition is symbolically shown on the display 15. When the hand unit 1 is connected to the docking station 3, the battery 17 is automatically connected to a power unit 29 which via mains or other sources will supply it with necessary energy to manage required number of breath analyses until next docking. At docking, connection between the microprocessor 18 and another microprocessor 30 in the docking station is established. Then more extensive function control is executed along with data communication providing backup of information stored in the local memory of the hand unit.

It should be noted that the various components and blocks of figure 2 are not drawn according to their physical size. The hand unit 1 preferably has a volume of 500 ml or less to meet the demands on simple use. The hand unit 1 is preferably designed for single handed use made possible by the fact that the start button 23 only is required for operating the unit. Presetting, programming, calibration and other adjustments are preferably performed when the unit is in the docking condition, and connected to a computer.

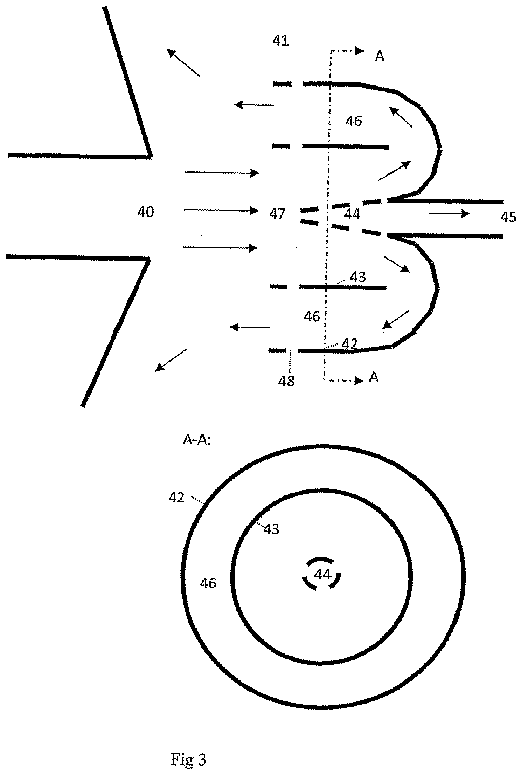

Figure 3 schematically shows a preferred embodiment of the receptor according to the invention. In this embodiment the receptor is coaxially divided into one inner inlet channel and one outer outlet channel with reverse flow direction.

From the mouth or nose opening 40 a flow of expiratory air is drawn as three arrows pointing to the right direction. The receptor 41 has been positioned adjacent to, and at a relatively close distance from the nose/mouth opening 40, without necessarily touching it or any other body part. The receptor 41 has an outer wall 42 which together with the opening towards the mouth/nose opening 40 defines a certain volume, the size of which does not exceed the desired sample volume. Furthermore, the receptor 41 includes an inner wall 43 which like the outer wall 42 opens itself towards the mouth/nose opening 40. The opening of the inner wall 43 preferably has an area which is entirely enclosed by the opening of the outer wall 42. The walls 42 and 43 are fixed against each other with pins or correspondingly, not shown in the figure, and which have minimum flow resistance. Seen from the mouth/nose opening 40 the receptor 41 has two concentric openings. That the walls 42 and 43 have been drawn with circular cross section should be seen as examples of design without preference. Other shapes are possible and may be advantageous.

The opening 47 of the receptor 41 is preferably designed such that it does not by accidence block the mouth/nose opening 40 and thereby preventing the test person from breathing. The walls 42 and 43 preferably are equipped with one or several side holes 48 for that purpose, having a total cross section area of at least 1 cm². Along the symmetry axis of the receptor 41, there is a receiving tube 44 with connection 45 to the measuring cell of the apparatus.

Respiratory air from the mouth/nose opening 40 is incident through the opening 47 of the inner wall 43 and is then distributed into one branch through the receiving tube 44 to the measuring cell, and another branch following the curvature of the outer wall 42, the shape of which leads the air flow backwards in the opposite direction to the incident air flow. The inner wall 43 in combination with the curvature of the outer wall 42 prevents eddy formation and turbulence. The opening 47 and the intermediate air layer 46 define an inner and outer flow channel with opposite flow directions.

The method and apparatus according to the invention can be varied in many ways within the scope of the claims described below.

## Claims

1. Method for breath analysis comprising
providing a measuring cell (4) comprising a receptor (2; 41) having an inlet opening (24; 47) and IR detectors (8, 9) provided in the cell;
determining the concentration of a volatile substance within said measuring cell;
sampling a breath sample from a test person in order to receive a flow of expired air from the test person by air intake to said measuring cell through the inlet opening of the receptor;
positioning the measuring cell adjacent the mouth and nose opening of the test person without physical contact between said receptor and said test person; wherein said receptor has the shape of a cup, funnel or scoop, which has a geometry so as to causes a reflow of a part of the expired air protecting the breath sample from being mixed with ambient air thereby preserving the concentration of the volatile substance in the expired air; and
measuring the concentration of a physiological reference substance, simultaneously with the determination of the concentration of the volatile substance, within said measuring cell, and if the concentration is lower than the normal concentration value of said substance, the sample has been diluted with ambient air, whereby a quality measure of said first determination and of said concentration preserving effect is provided.

2. Method according to claim 1, wherein the physiological reference substance is CO₂ and the concentration is determined by the partial pressure thereof, the normal value being the partial pressure in the inner airways.

3. Method according to claim 1, wherein the physiological reference substance is water vapour and the concentration is determined by the partial pressure thereof, the normal value being the partial pressure in the inner airways.

4. Method according to claim 1, further comprising the operator manually giving a starting command for breath sampling, controlling duration of air intake by a sensor signal from said measuring cell (4), presenting result during or immediately after finished sampling, and stopping air flow at sampling and zeroing before analysis of said substance.

5. Method according to claim 1, wherein sampling comprises a volume and air flow not exceeding 50 ml and 10 ml per second, respectively, at insignificant flow resistance, sampling being essentially contactless, and air intake is controlled by a pumping element (16).

6. Method according to claim 1, wherein zeroing and function test is automatically executed and includes control with respect to absolute value of time variation of measuring variable, and presence of receptor (2; 41).

7. Method according to claim 1, wherein said determination is performed by IR spectroscopic analysis, and includes the presence of other volatile organic substance than ethyl alcohol within said breath sample.

8. Method according to claim 1, wherein the timing of sampling is controlled by a starting command from operator, and the duration of air intake is controlled by sensor signal from the measuring cell.

9. A breath analyzer comprising a measuring cell (4), a receptor (2, 41) connected to said measuring cell and including an inlet opening (24) adapted to receive expired air from the mouth and nose of a test person, and a connector (45) coupled to the measuring cell (4); wherein the receptor (2, 41) has the shape of a cup, funnel or scoop with a geometry adapted to create a reflow back to said inlet opening (24) of part of the expired air received at said inlet opening (24) and part of which is fed to said measuring cell, said measuring cell including an IR source (10), and IR detectors (13, 14) adapted for measurement of the concentrations of both a volatile substance, and a physiological reference substance.

10. The breath analyzer according to claim 9, wherein the receptor (2, 41) has a partly confined inner volume which is smaller than a test person's tidal volume.

11. The breath analyzer according to claim 9 or 10, wherein the receptor is adapted to distribute flow of air into one branch flowing into the measuring cell where the breath analysis takes place, and another branch which is redirected to the inlet opening, whereby the reflow branch forms a protective curtain, preventing the incident flow from mixing with ambient air.

12. Breath analyzer according to claim 9, wherein said receptor (2;41) is exchangeable and includes element for separation of liquid drops or particles from said breath sample, and mechanical support element for fixation to a body part, e g the nose and chin tips.

13. Breath analyzer according to claim 9, further including a single-hand operated hand unit 1, an exchangeable receptor (2;41) and a docking station (3) for placement of said hand unit (1) when inactivated.

## Patentansprüche

1. Verfahren zur Atmungsanalyse, umfassend
Bereitstellen einer Messzelle (4), die einen Rezeptor (2; 41) mit einer Einlassöffnung (24; 47) und in der Zelle vorgesehene IR-Detektoren (8, 9) umfasst;
Bestimmen der Konzentration einer flüchtigen Substanz innerhalb der Messzelle; Probenahme einer Atemprobe von einer Testperson, um einen Strom von Luftabgabe von der Testperson durch Luftaufnahme zu der Messzelle durch die Einlassöffnung des Rezeptors zu erhalten;
Positionieren der Messzelle angrenzend an die Mund- und Nasenöffnung der Testperson ohne physischen Kontakt zwischen dem Rezeptor und der Testperson; wobei der Rezeptor die Form eines Bechers, Trichters oder einer Schaufel aufweist, eine Geometrie aufweisend, die einen Rückstrom eines Teils der Luftabgabe bewirkt, der die Atemprobe vor dem Vermischen mit Umgebungsluft schützt, um dadurch die Konzentration der flüchtigen Substanz in der Luftabgabe zu bewahren; und
Messen der Konzentration einer physiologischen Referenzsubstanz gleichzeitig mit dem Bestimmen der Konzentration der flüchtigen Substanz innerhalb der Messzelle, und wenn die Konzentration niedriger als der normale Konzentrationswert der Substanz ist, wurde die Probe mit Umgebungsluft verdünnt, wodurch ein Qualitätsmaß der ersten Bestimmung und der konzentrationserhaltenden Wirkung bereitgestellt wird.

2. Verfahren nach Anspruch 1, wobei die physiologische Referenzsubstanz CO₂ ist und die Konzentration durch den Partialdruck davon bestimmt wird, wobei der Normalwert der Partialdruck in den inneren Atemwegen ist.

3. Verfahren nach Anspruch 1, wobei die physiologische Referenzsubstanz Wasserdampf ist und die Konzentration durch den Partialdruck davon bestimmt wird, wobei der Normalwert der Partialdruck in den inneren Atemwegen ist.

4. Verfahren nach Anspruch 1, weiter umfassend, dass der Bediener manuell einen Startbefehl für die Atemprobenahme abgibt, die Dauer der Luftaufnahme durch ein Sensorsignal von der Messzelle (4) steuert, das Ergebnis während oder unmittelbar nach der abgeschlossenen Probenahme präsentiert und den Luftstrom bei der Probenahme anhält und vor der Analyse der Substanz Null einstellt.

5. Verfahren nach Anspruch 1, wobei die Probenahme einen Volumen- und Luftstrom umfasst, der 50 ml bzw. 10 ml pro Sekunde bei unbedeutendem Strömungswiderstand nicht überschreitet, wobei die Probenahme im Wesentlichen berührungslos ist und die Luftaufnahme durch ein Pumpelement (16) gesteuert wird.

6. Verfahren nach Anspruch 1, wobei Nulleinstellung und Funktionstest automatisch ausgeführt wird und eine Steuerung in Bezug auf den Absolutwert der Zeitänderung der Messgröße und das Vorhandensein des Rezeptors (2; 41) beinhaltet.

7. Verfahren nach Anspruch 1, wobei die Bestimmung durch IR-Spektroskopieanalyse durchgeführt wird und das Vorhandensein einer anderen flüchtigen organischen Substanz als Ethylalkohol in der Atemprobe beinhaltet.

8. Verfahren nach Anspruch 1, wobei der Zeitpunkt der Probenahme durch einen Startbefehl des Bedieners gesteuert wird und die Dauer der Luftaufnahme durch ein Sensorsignal der Messzelle gesteuert wird.

9. Atemanalysator, umfassend eine Messzelle (4), einen mit der Messzelle verbundenen Rezeptor (2, 41) und enthaltend eine Einlassöffnung (24) zum Aufnehmen von Luftabgabe aus dem Mund und der Nase einer Testperson und einen mit der Messzelle (4) gekoppelten Verbinder (45);
wobei der Rezeptor (2, 41) die Form eines Bechers, Trichters oder einer Schaufel mit einer Geometrie aufweist, die angepasst ist, um einen Rückstrom zu der Einlassöffnung (24) eines Teils der an der Einlassöffnung (24) aufgenommenen Luftabgabe zu erzeugen und von der ein Teil der Messzelle zugeführt wird, wobei die Messzelle eine IR-Quelle (10) und IR-Detektoren (13, 14) beinhaltet, die zum Messen der Konzentrationen sowohl einer flüchtigen Substanz als auch einer physiologischen Referenzsubstanz angepasst sind.

10. Atemanalysator nach Anspruch 9, wobei der Rezeptor (2, 41) ein teilweise begrenztes Innenvolumen aufweist, das kleiner ist als das Tidalvolumen einer Testperson.

11. Atemanalysator nach Anspruch 9 oder 10, wobei der Rezeptor angepasst ist, um den Luftstrom in einen Zweig zu verteilen, der in die Messzelle strömt, in der die Atmungsanalyse stattfindet, und in einen weiteren Zweig, der zur Einlassöffnung umgeleitet wird, wobei der Rückstromzweig einen Schutzvorhang bildet, der verhindert, dass sich der einfallende Strom mit der Umgebungsluft vermischt.

12. Atemanalysator nach Anspruch 9, wobei der Rezeptor (2; 41) austauschbar ist und ein Element zum Trennen von Flüssigkeitstropfen oder -teilchen aus der Atemprobe und ein mechanisches Trägerelement zum Fixieren an einem Körperteil, z.B. der Nase und den Kinnspitzen, beinhaltet.

13. Atemanalysator nach Anspruch 9, der weiter eine einhändig bedienbare Handeinheit (1), einen austauschbaren Rezeptor (2; 41) und eine Dockingstation (3) zum Platzieren der Handeinheit (1) bei Inaktivierung enthält.

## Revendications

1. Procédé d'analyse respiratoire comprenant
la fourniture d'une cellule de mesure (4) comprenant un récepteur (2 ; 41) ayant une ouverture d'entrée (24 ; 47) et des détecteurs IR (8, 9) fournis dans la cellule ;
la détermination de la concentration d'une substance volatile à l'intérieur de ladite cellule de mesure ;
l'échantillonnage d'un échantillon respiratoire sur une personne à tester de manière à recevoir un flux d'air expiré de la personne à tester par absorption d'air dans ladite cellule de mesure à travers l'ouverture d'entrée du récepteur ;
le positionnement de la cellule de mesure à côté de l'ouverture de la bouche et du nez de la personne à tester sans contact physique entre ledit récepteur et ladite personne à tester ; dans lequel ledit récepteur a la forme d'une coupelle, d'un entonnoir ou d'une cuillère qui a une géométrie telle qu'elle entraîne un reflux d'une partie de l'air expiré empêchant le mélange de l'échantillon respiratoire avec l'air ambiant et préservant ainsi la concentration de la substance volatile dans l'air expiré ; et
la mesure de la concentration d'une substance physiologique de référence simultanément à la détermination de la concentration de la substance volatile, à l'intérieur de ladite cellule de mesure, et si la concentration est inférieure à la valeur de concentration normale de ladite substance, l'échantillon a été dilué avec de l'air ambiant, moyennant quoi une mesure de qualité de ladite première détermination et dudit effet de préservation de la concentration est fournie.

2. Procédé selon la revendication 1, dans lequel la substance de référence physiologique est le CO₂ et la concentration est déterminée par sa pression partielle, la valeur normale étant la pression partielle dans les voies respiratoires intérieures.

3. Procédé selon la revendication 1, dans lequel la substance de référence physiologique est la vapeur d'eau et la concentration est déterminée par sa pression partielle, la valeur normale étant la pression partielle dans les voies respiratoires intérieures.

4. Procédé selon la revendication 1, comprenant en outre la commande manuelle de démarrage de l'échantillonnage respiratoire par l'opérateur, la commande de la durée d'absorption d'air par un signal de capteur de ladite cellule de mesure (4), la présentation du résultat pendant ou immédiatement après la fin de l'échantillon, et l'arrêt du flux d'air au moment de l'échantillonnage et la mise à zéro avant l'analyse de ladite substance.

5. Procédé selon la revendication 1, dans lequel l'échantillonnage comprend un volume et un flux d'air n'excédant pas 50 ml et 10 ml par seconde, respectivement, à une résistance au flux insignifiante, l'échantillonnage étant fondamentalement sans contact, et l'absorption d'air est commandée par un élément de pompage (16).

6. Procédé selon la revendication 1, dans lequel une remise à zéro et un test des fonctions sont automatiquement exécutés et comprennent une commande relativement à une valeur absolue de variation temporelle de la variable de mesure, et la présence d'un récepteur (2 ; 41).

7. Procédé selon la revendication 1, dans lequel ladite détermination est réalisée par analyse spectroscopique IR, et comprend la présence d'une substance organique volatile autre quel l'alcool éthylique dans ledit échantillon respiratoire.

8. Procédé selon la revendication 1, dans lequel la temporisation de l'échantillonnage est commandée par une commande de démarrage issue par un opérateur, et la durée d'absorption d'air est commandée par un signal de capteur de la cellule de mesure.

9. Analyseur respiratoire comprenant une cellule de mesure (4), un récepteur (2, 41) raccordé à ladite cellule de mesure et comprenant une ouverture d'entrée (24) adaptée à recevoir l'air expiré de la bouche et du nez d'une personne à tester, et un raccord (45) couplé à la cellule de mesure (4) ;
dans lequel
le récepteur (2, 41) a la forme d'une coupe, d'un entonnoir ou d'une cuillère avec une géométrie adaptée pour créer un reflux qui retourne dans ladite ouverture d'entrée (24) d'une partie de l'air expiré reçu au niveau de ladite ouverture d'entrée (24) et dont une partie est alimentée à ladite cellule de mesure, ladite cellule de mesure comprenant une source d'IR (10), et des détecteurs d'IR (13, 14) adaptés à mesurer les concentrations d'une substance volatile, et d'une substance physiologique de référence.

10. Analyseur respiratoire selon la revendication 9, dans lequel le récepteur (2, 41) a un volume intérieur partiellement confiné qui est plus petit qu'un volume respiratoire d'une personne à tester.

11. Analyseur respiratoire selon la revendication 9 ou 10, dans lequel le récepteur est adapté à distribuer un flux d'air dans une branche s'écoulant dans la cellule de mesure où l'analyse respiratoire a lieu, et une autre branche qui est redirigée vers l'ouverture d'entrée, moyennant quoi la branche de reflux forme un rideau de protection, empêchant le flux incident de se mélanger avec l'air ambiant.

12. Analyseur respiratoire selon la revendication 9, dans lequel ledit récepteur (2 ; 41) est échangeable et comprend un élément pour la séparation de particules ou gouttes de liquide dudit échantillon respiratoire, et un élément de support mécanique pour la fixation à une partie du corps, par exemple à la pointe du nez et à la pointe menton.

13. Analyseur respiratoire selon la revendication 9, comprenant en outre une unité manuelle actionnée d'une seule main(1), un récepteur échangeable (2 ; 41) et une station d'accueil (3) pour le positionnement de ladite unité manuelle (1) lorsqu'elle est inactive.
